# EUROPÄISCHE PATENTSCHRIFT

(11) **EP 2 735 291 B1**
(45) Veröffentlichungstag und Bekanntmachung des Hinweises auf die Patenterteilung: **22.05.2019**
(21) Anmeldenummer: 13194458.9
(22) Anmeldetag: 26.11.2013
(51) Int. Cl.: A61F 5/01, A61F 5/03, A61F 2/50, A61F 2/68, A61N 2/02, A61N 2/06, A61N 1/04, A61N 1/36, A61F 7/00, A61H 23/02, A61H 39/00, A61H 39/04, A61H 39/06, A61N 2/00, A61F 2/78, A61F 2/80

(54) **Prothese oder Orthese**
Prosthesis or orthosis
Prothèse ou orthèse

(30) Priorität: 26.11.2012 DE 102012023070
(43) Veröffentlichungstag der Anmeldung: 28.05.2014
(73) Patentinhaber: Pohlig GmbH, 83278 Traunstein (DE)
(72) Erfinder: Pohlig, Kurt, 83278 Traunstein (DE); Schäfer, Michael, 83278 Traunstein (DE)
(74) Vertreter: Pfenning, Meinig & Partner mbB

(56) Entgegenhaltungen:
- WO-A1-96/32909
- WO-A1-99/61103
- WO-A1-2012/069923
- WO-A2-01/10370
- WO-A2-2007/102156
- US-A- 5 108 456
- US-A1- 2002 099 450
- US-A1- 2011 118 853

## Beschreibung

Die Erfindung betrifft eine Prothese oder Orthese für den Ersatz von äußeren Körperteilen oder Gliedmaßen, beispielsweise nach Amputationen, bzw. zum Stabilisieren, Entlasten, Ruhigstellen und Führen von Gliedmaßen oder des Rumpfs oder zum Korrigieren von Fehlstellungen von Gliedmaßen oder des Rumpfs.

In der traditionellen chinesischen Medizin nehmen die Akupunktur und die Akupressur einen großen Raum ein. Die Akupunktur beinhaltet das Stechen mit Nadeln in Akupunkturpunkte der Haut zur Behandlung von Krankheiten und zur Schmerztherapie. Derartige Akupunkturpunkte sind über den gesamten Körper verteilt entlang den sogenannten "Meridianen" zu finden. Die Akupressur ist eine verwandte Methode der Akupunktur, bei der ein stumpfer Druck auf die auf den Meridianen liegenden Akupunkturpunkte ausgeübt wird. Im Fall der Akupressur spricht man dann auch von Akupressurpunkten. Werden die Akupressurpunkte erwärmt, handelt es sich um die Moxibustionstherapie. Im Folgenden werden unter Akupunkturpunkt, Akupressurpunkt oder Moxibustionspunkt jeweils dasselbe verstanden, so daß diese Bezeichnungen dieselben Punkte bezeichnen.

Akupressur wird zur Behandlung verschiedener chronischer Krankheiten und Schmerzen angewandt. Die Behandlungsdauer beschränkt sich dabei meist auf einzelne Therapiesitzungen und reicht oftmals nicht aus, um chronische Krankheiten oder hochspezifische akute und chronische Schmerzen wirksam zu therapieren.

Die Druckschrift WO 2012/069923 A1 offenbart eine Lendenstützvorrichtung, welche ein Basiselement und ein Polsterelement, das an dem Basiselement befestigt ist, beinhaltet, wobei das Polsterelement eine Vielzahl von Druckelementen, die sich von einem vorderen Abschnitt des Basiselements erstrecken. Die Lendenstützvorrichtung ist geeignet, um zwischen der Kleidung eines Benutzers und dem Rücken eines Benutzers positioniert zu werden, ohne eine Befestigung an der Kleidung des Benutzers oder dem Rücken des Benutzers, so dass die Vielzahl von Druckelementen mit mehreren diskreten Stellen auf dem Rücken des Benutzers in Eingriff sind.

Die Druckschrift WO 96/32909 A1 offenbart ein Verfahren und eine Vorrichtung zum Steuern einer Reflexantwort der Muskeln eines Körpergelenks, wobei ein externer Druck auf die Mechanorezeptoren angewendet wird, welche die natürlichen Rezeptoren und die tieferen Gelenk-Mechanorezeptoren führen.

Die Druckschrift US 2002/0099450 A1 offenbart ein dynamisches Befestigungssystem mit variabler Geometrie für einen Prothesenschaft. Das System verfügt über flüssigkeitsgefüllte Blasen, deren Volumina sich automatisch regulierbaren, um einen kontinuierlich sicheren Sitz des Prothesenschafts zu gewährleisten. Das Befestigungssystem ist in der Lage das Potenzial für Gewebeläsionen zu reduzieren.

Ausgehend hiervon ist es daher die Aufgabe der vorliegenden Erfindung eine Prothese oder Orthese bereitzustellen, die zur Behandlung hochspezifischer akuter und chronischer Schmerzen, die auch aus der Verwendung einer Prothese oder Orthese resultieren können, sowie der hierfür verantwortlichen Auslöser geeignet ist. Ferner soll eine Prothese oder Orthese bereitgestellt werden, die es ermöglich, das Problem akuter und chronischer Schmerzen in einer für den Patienten akzeptablen Art und Weise zu lösen.

Die Aufgabe wird durch eine Prothese oder Orthese nach Anspruch 1 gelöst. Vorteilhafte Ausgestaltungen der erfindungsgemäßen Prothese oder Orthese sind in den abhängigen Ansprüchen beschrieben. Die erfindungsgemäße Prothese oder Orthese weist einen einschaligen oder mehrschaligen Schaft zur Aufnahme eines Körperteils auf, wobei der Schaft eine innere Wandung aufweist, die
einen Körperteilaufnahmebereich begrenzt. Unter dem Körperteilaufnahmebereich ist die Ausnehmung des Schaftes zu verstehen, die von der inneren Wandung begrenzt wird. Der Schaft ist mit dieser Ausnehmung an ein Körperteil ansteckbar oder anlegbar, wobei die innere Wandung am Körperteil anliegt.

Die innere Wandung umschließt den Körperteilaufnahmebereich in mindestens einer Ebene vollständig.

Die innere Wandung der erfindungsgemäßen Prothese oder Orthese weist außerdem mindestens eine Ausstülpung, vorzugsweise mindestens zwei Ausstülpungen, auf, die entlang eines Akupunktur-Meridians (im Folgenden auch Meridian genannt) und/oder entlang einer Nervenbahn angeordnet ist bzw. angeordnet sind. Eine derartige Ausstülpung wird im Folgenden auch Druckpunkt oder auch Akupressor genannt, kann jedoch konvex oder konkav ausgestaltet sein.

Die derart ausgestaltete erfindungsgemäße Prothese oder Orthese ermöglicht eine Stimulation eines Akupressurpunktes entlang eines Meridians im Bereich des vom Schaft aufgenommenen Körperteils. Die Integration der Akupressur in eine Prothese oder eine Orthese ermöglicht eine Akupressurbehandlung mit einer besonders langen Therapiedauer. Bei einer durchschnittlichen Tragezeit von über zwölf Stunden ergibt sich hierbei eine herausragende Möglichkeit einer dauerhaften Einflussnahme auf chronische und akute Schmerzen. Dies ist gleichermaßen vorteilhaft für die Behandlung des Phantomschmerzes als auch von Schmerzen im Sinne einer Befindlichkeitsstörung.

Die Ausstülpungen weisen dabei vorzugsweise eine Tiefe von 5 mm ± 3 mm und einen mittleren Flächendurchmesser von 50 mm ± 10 mm auf. Unter der Tiefe der Ausstülpung wird die maximale Ausdehnung der Ausstülpung radial zur Längsachse des Schafts gemessen von der Oberfläche der inneren Wandung verstanden. Unter dem Flächendurchmesser der Ausstülpung wird die Ausdehnung der Ausstülpung entlang der Oberfläche der inneren Wandung verstanden.

Alternativ kann die innere Wandung auch eine Vielzahl von Ausstülpungen aufweisen, die entlang eines Meridians und/oder entlang einer Nervenbahn, vorzugsweise alle und/oder ausschließlich entlang eines Meridians und/oder entlang einer Nervenbahn angeordnet sind. Eine größere Anzahl solcher Akupressoren verstärkt die Stimulation des stimulierten Meridians bzw. die Akupressurwirkung.

Ferner ist es denkbar die Ausstülpungen entlang verschiedener Meridiane anzuordnen, um somit gleichzeitig mehrere Meridiane stimulieren zu können. Hierdurch kann eine Optimierung des Therapieergebnisses verschiedener Beeinträchtigungen erreicht werden.

Die Ermittlung der einzelnen Akupressurpunkte erfolgt mittels eines hierfür speziell entwickelten Gerätes, wodurch die Lokalisation auf den einzelnen Meridianen gut erfolgen kann.

Die mindestens eine Ausstülpung kann als Auskragung oder als Vertiefung ausgebildet sein. Bei einer Vielzahl von Ausstülpungen ist es auch denkbar, dass ein Teil der Ausstülpungen als Auskragungen ausgebildet ist und ein weiterer Teil der Ausstülpungen als Vertiefungen ausgebildet ist. In den Ausstülpungen, die als Vertiefungen ausgebildet sind, kann sich ein Unterdruck einstellen. Hierbei erfolgt die Stimulation mittels einer lokal auf einen Druckpunkt aufgebrachten Zugkraft. Die Ausstülpungen, die als Auskragungen ausgebildet sind, üben eine Druckkraft auf einen Druckpunkt aus.

Der Schaft der Prothese oder Orthese kann vorzugsweise zylinderförmig oder konisch gestaltet sein. Die Ausstülpungen können kuppelförmig, kantig, konvex, konkav, sternförmig, oval oder eckig ausgebildet sein.

Eine besonders vorteilhafte Ausgestaltung der Erfindung liegt darin, die Tiefe und/oder die Dicke bzw. die Gesamtgröße einer Ausstülpung oder einer Vielzahl von Ausstülpungen pneumatisch oder hydraulisch oder mechanisch variierbar zu gestalten. Hierdurch kann die Prothese oder Orthese an kurzfristige oder langfristige Veränderungen des Umfangs des Körperteils, z.B. durch tageszeitliches An- und Abschwellen des Körperteils oder durch Gewichtszunahme und -abnahme, optimal angepasst werden, um die Druck- bzw. Zugkraft der Ausstülpungen konstant zu halten.

Zu diesem Zweck kann im Schaft ein System miteinander kommunizierender Röhren integriert sein, das die Tiefe und/oder Dicke bzw. die Gesamtgröße der Ausstülpungen hydraulisch oder pneumatisch oder mechanisch reguliert. Mit einem solchen System kann das Schaftvolumen auch derart geregelt werden, dass beispielsweise bei punktuell starkem Druck ausgehend von einer ersten Ausstülpung, beispielsweise beim Sitzen mit einer Beinprothese oder - orthese, dieser auf andere Ausstülpungen, die mit der ersten Ausstülpung über das Röhrensystem verbunden sind, verlagert werden kann. Dieses Röhrensystem ist insbesondere dazu geeignet, bei einer Vielzahl von Ausstülpungen die Wirkung der Druckpunkte unterschiedlich intensiv zu gestalten.

Alternativ ist es auch möglich, dass zur Regulierung der Tiefe und/oder der Dicke bzw. der Gesamtgröße einer Ausstülpung oder einer Vielzahl von Ausstülpungen ein Wechseldrucksystem vorgesehen ist, womit die Tiefe und/oder Dicke bzw. die Gesamtgröße von einzelnen oder mehreren Ausstülpungen abwechselnd erhöht oder verringert werden kann. Ein derartiges Wechseldrucksystem ist insbesondere geeignet, bei einer Vielzahl von Druckpunkten die Wirkung der Druckpunkte unterschiedlich zu gestalten und zeitlich zu verändern, z.B. wenn Druckstellen vermieden werden sollen.

Eine weitere vorteilhafte Ausgestaltung sieht vor, dass mindestens eine Ausstülpung mindestens eine Kammer oder Hohlraum aufweist. Diese Kammer oder Hohlraum ist geeignet zusätzliche Instrumente oder Mittel zur Überwachung oder Behandlung des Körperteils aufzunehmen. Ebenso können auch mehrere oder alle Ausstülpungen eine oder mehrere Kammern aufweisen.

Beispielsweise kann in mindestens einer Kammer ein Permanentmagnet oder ein Elektromagnet zur Erzeugung eines statischen oder eines gepulsten Magnetfeldes angeordnet sein. Gepulste elektromagnetische Felder (PEMF) sollen bei bestimmten Frequenzen den Organismus bioenergetisch positiv beeinflussen und die Durchblutung fördern, um den Zellstoffwechsel anzuregen.

Eine weitere Möglichkeit sieht vor, dass mindestens eine Kammer ein Piezoelement zur elektrischen und/oder mechanischen Stimulation des Körperteils beinhaltet.

In mindestens einer Kammer mindestens einer Ausstülpung können alternativ auch Medikamente deponiert werden, wobei die Medikamente über eine oder mehrere Öffnungen, Poren oder eine Perforation in der Kammer an eine Oberfläche des in dem Schaft angeordneten Körperteils abgebbar sind. Gleichermaßen ist auch die Einrichtung mehrerer verschiedener Medikamentendepots in verschiedenen Kammern einer oder mehrerer Ausstülpungen möglich, wenn mehr als ein Medikament verabreicht werden soll.

In einer weiteren vorteilhaften Ausgestaltung der Erfindung sind eine oder mehrere Ausstülpungen zur Beheizung und/oder Kühlung des Schafts eingerichtet. Durch die Zufuhr von Wärme werden beispielsweise die Gefäße erweitert und die Durchblutung gefördert. Zur Beheizung und/oder Kühlung kann der Schaft von einem Schlauchsystem durchzogen sein, das mit den Ausstülpungen wärmeleitend verbunden ist. Alternativ können eine oder mehrere Kammern einer oder mehrerer Ausstülpungen als Wärmekammer zur Erwärmung von arteriellem Blut an einer zentralen Einströmstelle des arteriellen Blutes (z.B. Arteria femoralis) und /oder als Kühlkammer zur Abkühlung arteriellen Blutes eingerichtet sein, um das peripher strömende Blut zu erwärmen bzw. abzukühlen und so beispielsweise dem oft beklagten Kältegefühl oder einem unangenehmen Wärmegefühl im Beinstumpf entgegenzuwirken.

Ferner kann mindestens eine Kammer ein Vibrationselement beinhalten. Das Vibrationselement überträgt mechanische Schwingungen auf das von der Prothese oder Orthese aufgenommene Körperteil. Dies fördert die Relaxierung der Muskulatur und die Durchblutung des Körperteils und reduziert Muskelverspannungen.

In einer weiteren möglichen Ausgestaltung der Erfindung kann mindestens eine Kammer mindestens einer Ausstülpung eine Füllung mit Feuchtigkeit aufsaugendem Material zur Dehydrierung des in der Regel sehr feuchten Innenschaftklimas aufweisen. Dieses Feuchtigkeit aufsaugende Material kann beispielsweise in Form von Pads in eine oder mehrere Kammern einer oder mehrerer Ausstülpungen eingesetzt sein. Alternativ kann in einer oder mehreren Kammern einer oder mehrerer Ausstülpungen auch ein elektronisch und/oder mechanisch wirkendes Entfeuchtungselement angeordnet sein.

In einer weiteren vorteilhaften Ausgestaltung der Erfindung weist mindestens eine Kammer in mindestens einer Ausstülpung mindestens ein Messgerät zur Messung von Körperzuständen, eines Blutdrucks, einer Strömungsgeschwindigkeit von Blut und/oder einer Körpertemperatur auf.

Darüberhinaus ist für Langzeitdiagnosen die Abfrage von bionischen Daten interessant. Hierzu kann mindestens eine Kammer mindestens einer Ausstülpung ein Messgerät zur Erfassung von bionischen Daten wie z.B. eines Oberflächendruckes an definierten Messpunkten, einer Schaftinnentemperatur und/oder eines Feuchtigkeitsgrades aufweisen.

In einer weiteren vorteilhaften Ausgestaltung der Erfindung kann mindestens in einer Ausstülpung eine Elektrode zur Abnahme von myoelektrischen Impulsen zur Steuerung von mechanischen Knie-, Fuß-, Hand- oder Ellenbogengelenken angebracht sein.

Zur Fern-Überwachung der Rehabilitationstherapie oder der Tragezeit kann mindestens eine der Ausstülpungen auch mindestens einen Temperatur- und/oder Bewegungslogger aufweisen.

Die Ausstülpungen und die innere Wandung weisen vorteilhafterweise das gleiche Material auf. Je nach Versorgungsziel kann die innere Wandung beispielsweise ein elastisches Material, ein Material mit einem weichen Shore-Grad oder ein rigides Material aufweisen, in das die Ausstülpungen integriert sind.

Die Oberfläche der Ausstülpungen kann glatt, rau, adhäsiv, mit einem Relief und/oder mit Noppen versehen sein. Die Noppen können vollständig mit Material gefüllt oder hohl gestaltet sein.

Es ist ebenso denkbar, dass die Ausstülpungen und die innere Wandung unterschiedliche Materialien aufweisen. In diesem Fall können die Ausstülpungen vorzugsweise auf eine Oberfläche der inneren Wandung aufgeklebt sein.

Des Weiteren können die Ausstülpungen auch mit Pelotten gefüllt sein.

Um eine oder mehrere Ausstülpungen nach Fertigstellung des Schafts der Prothese oder Orthese noch umpositionieren zu können, sind die Ausstülpungen auch mobil oder temporär adaptierbar an der inneren Wandung angebracht sein.

Eine weitere vorteilhafte Ausgestaltung der Erfindung sieht die Integration eines TENS-Geräts zur Schmerzbehandlung an den Extremitäten in die Prothese oder Orthese vor. Die TENS-Geräte geben über Elektroden einen Reizstrom an die Haut ab. Eine solche TENS-Elektrode kann beispielsweise in einer Kammer einer Ausstülpung angeordnet sein. Das Therapieziel ist die Schmerzreduktion durch Verringerung oder Verhinderung der Schmerzweiterleitung an das Gehirn. Ein derartiges TENS-Gerät wird beispielsweise bei der Behandlung des Phantomschmerzes angewandt.

Eine weitere vorteilhafte Erfindung sieht vor, dass mindestens eine Kammer mindestens einer Ausstülpung einen Sensor für eine funktionelle Elektrostimulation, z.B. zur Behandlung nach einem Schlaganfall, aufweist.

Eine besonders vorteilhafte Ausgestaltung der Erfindung kann auch darin bestehen, mindestens eine der Ausstülpungen oder mindestens eine Kammer mindestens einer der Ausstülpungen mit verschiedenen Funktionen auszustatten. So kann mindestens eine Ausstülpung oder mindestens eine Kammer mindestens einer der Ausstülpungen eine Kombination der folgenden Merkmale aufweisen: einen Mechanismus zur Regulierung der Tiefe und/oder Dicke der Ausstülpungen, einen Permanentmagneten oder Elektromagneten, ein Piezoelement, ein Medikamentendepot, eine Heizung und/oder Kühlung, ein Vibrationselement, eine Füllung mit Feuchtigkeit aufsaugendem Material, ein Entfeuchtungselement, ein Messgerät zur Messung von Körperzuständen, ein Messgerät zur Messung von bionischen Daten, eine Elektrode zur Abnahme von myoelektrischen Impulsen, einen Temperaturlogger, einen Bewegungslogger, eine TENS-Elektrode. Dies ermöglicht eine gleichzeitige Behandlung verschiedener Beeinträchtigungen.

Für den Fall, dass die Ausstülpungen der Prothese oder Orthese für eine funktionelle Elektrostimulation ausgebildet sind, kann das Verfahren weiter dahingehend ausgestaltet sein, dass die Prothese oder Orthese ein elektrisches, mechanisches und/oder akustisches Signal aussendet, wenn die Prothese oder Orthese eine Bewegung des Körperteils registriert.

Des Weiteren umfasst die Verwendung der erfindungsgemäßen Prothese oder Orthese ihren Gebrauch als Arm- oder Beinprothese, als Arm- oder Beinorthese oder als Rumpfkorsett.

Nachfolgend werden einige vorteilhafte Ausführungsbeispiele einer erfindungsgemäßen Prothese oder Orthese anhand von Figuren genauer beschrieben. Die beschriebenen Merkmale sind jedoch nicht nur in Kombination der offenbarten Ausführungsbeispiele möglich, sondern können auch unabhängig vom konkreten Ausführungsbeispiel in verschiedenen Kombinationen realisiert sein. Es zeigen
- Figur 1: eine Seitenansicht einer Form für die Ausformung eines Beinprothesenschafts mit einer Vielzahl von Auskragungen,
- Figur 2: eine Seitenansicht einer Form für die Ausformung eines Beinprothesenschafts mit einer Vielzahl von Vertiefungen,
- Figur 3: einen einschaligen Prothesenschaft mit einer Einrichtung zur hydraulischen Regulierung der Tiefe der Auskragung,
- Figur 4: einen mehrschaligen Prothesenschaft mit einer Einrichtung zur hydraulischen Regulierung der Tiefe der Auskragung,
- Figur 5: einen Prothesenschaft mit einer Einrichtung zur mechanischen Regulierung der Tiefe der Auskragung,
- Figur 6: einen Prothesenschaft mit einem TENS-Gerät und
- Figur 7: eine schematische transparente Darstellung eines Oberschenkelstumpfes, in der der Ansatz einer Wärme- bzw. Kühlkammer gekennzeichnet ist.

Im Folgenden verweisen gleiche oder ähnliche Bezugszeichen auf gleiche oder ähnliche Elemente in den Figuren.

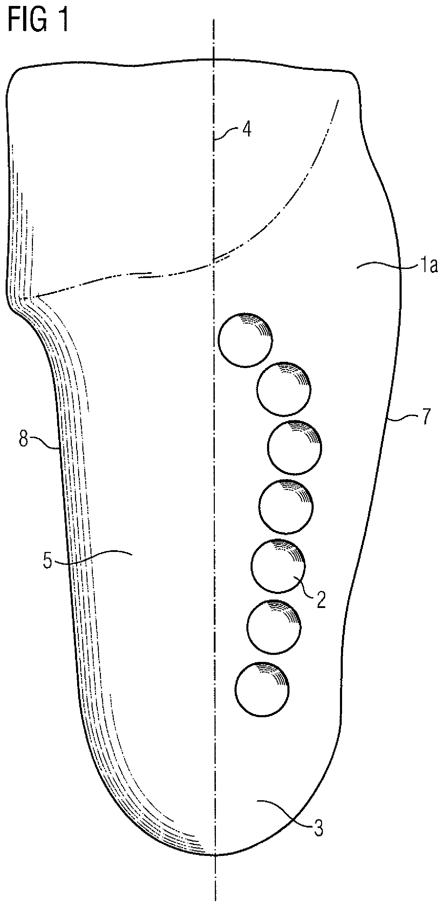

Figur 1 zeigt eine Seitenansicht einer Form 1a für die Ausformung einer inneren Wandung eines Schafts für eine Beinprothese mit einer Vielzahl von Auskragungen. Die Form 1a ist konisch ausgebildet und weist einen sich verjüngenden Teil 3 im unteren Bereich bezüglich einer Längsachse 4 der Form 1a auf. Des Weiteren weist die Form 1a eine Vorderseite 7 und eine Rückseite 8 auf. Im mittleren Bereich der Form 1a bezüglich der Längsachse 4 sind auf der Mantelfläche 5 sieben kuppelförmige Vertiefungen 2 angeordnet. Die Vertiefungen 2 reihen sich der Länge nach in etwa parallel zur Längsachse 4 aneinander. Dabei ist die Reihe der Vertiefungen 2 jeweils an Ihren Enden leicht zur Rückseite 8 hin gebogen.

Die Gestalt und Größe der Form 1a entspricht der Gestalt und Größe eines Oberschenkelstumpfes, der von dem mittels der Form 1a zu fertigenden Beinprothesenschaft (hier nicht gezeigt) aufgenommen werden kann. Zur Ausformung der inneren Wandung wird ein Schaftmaterial außen auf die Form 1a aufgebracht, sodass sich nach Entfernen der Form 1a ein zylindrischer Schaft (hier nicht gezeigt) mit einer konischen Ausnehmung in Gestalt der Form 1a ergibt. Diese konische Ausnehmung bildet einen Stumpfaufnahmebereich, dessen sich verjüngender Teil 3 der Prothese zugewandt ist. Der Stumpfaufnahmebereich wird von einer inneren Wandung des Schafts begrenzt. Die dem Stumpfaufnahmebereich zugewandte Oberfläche der inneren Wandung weist eine zur Reliefstruktur der Form 1a komplementäre Reliefstruktur auf. So entsprechen die kuppelförmigen Vertiefungen 2 der Mantelfläche 5 der Form 1a komplementären kuppelförmigen Auskragungen in der dem Stumpfaufnahmebereich zugewandten Oberfläche der inneren Wandung. Diese Ausstülpungen sind in Reihe längs eines Akupunkturmeridians eines durch den Stumpfaufnahmebereich aufzunehmenden Körperteils angeordnet und wirken daher als Akupressoren, die geeignet sind, eine Druckkraft auf Akupressurpunkte entlang eines Meridians auszuüben, der durch den von dem Beinprothesenschaft aufgenommenen Oberschenkel verläuft.

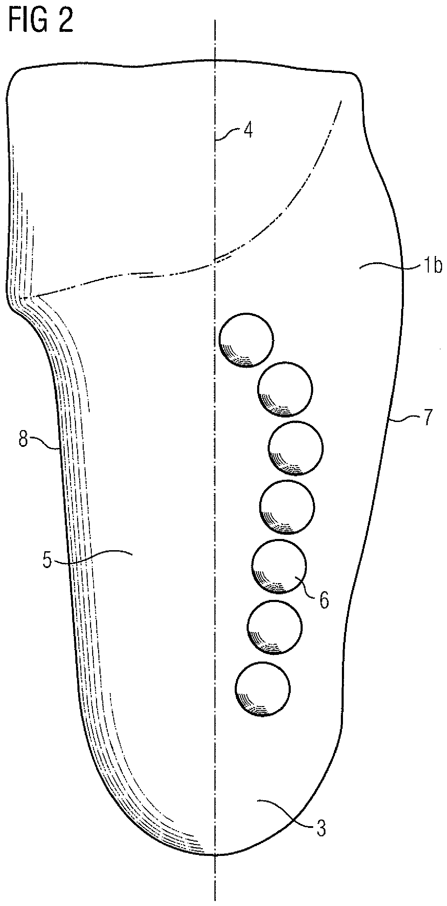

Figur 2 zeigt eine Seitenansicht einer weiteren Form 1b für die Ausformung einer inneren Wandung eines Schafts für eine Beinprothese mit einer Vielzahl an Auskragungen. Die Form 1b ist ähnlich wie die Form 1a in Figur 1 ausgebildet. Die Form 1b unterscheidet sich lediglich dadurch von der Form 1a, dass die Mantelfläche 5 der Form 1b in ihrem mittleren Bereich bezüglich der Längsachse 4 der Form 1b anstatt kuppelförmiger Vertiefungen 2 sieben in einer Reihe angeordnete kuppelförmige Auskragungen 6 aufweist. Die Reliefstruktur der Mantelfläche 5 der Form 1b ist komplementär zur Reliefstruktur einer einem Stumpfaufnahmebereich zugewandten Oberfläche einer mittels der Form 1b ausgeformten inneren Wandung eines Beinprothesenschafts (hier nicht gezeigt). So entsprechen die kuppelförmigen Auskragungen 6 in der Mantelfläche 5 der Form 1b komplementären kuppelförmigen Vertiefungen in einer einem Stumpfaufnahmebereich zugewandten Oberfläche einer mittels der Form 1b ausgeformten inneren Wandung eines Beinprothesenschafts. Diese Vertiefungen sind längs eines Akupunkturmeridians bzw. einer Nervenbahn eines durch den Stumpfaufnahmebereichs aufzunehmenden Körperteils angeordnet und wirken daher als Akupressoren, die geeignet sind, eine Zugkraft auf Akupressurpunkte entlang eines Meridians auszuüben, der durch den von dem Beinprothesenschaft aufgenommenen Oberschenkel verläuft.

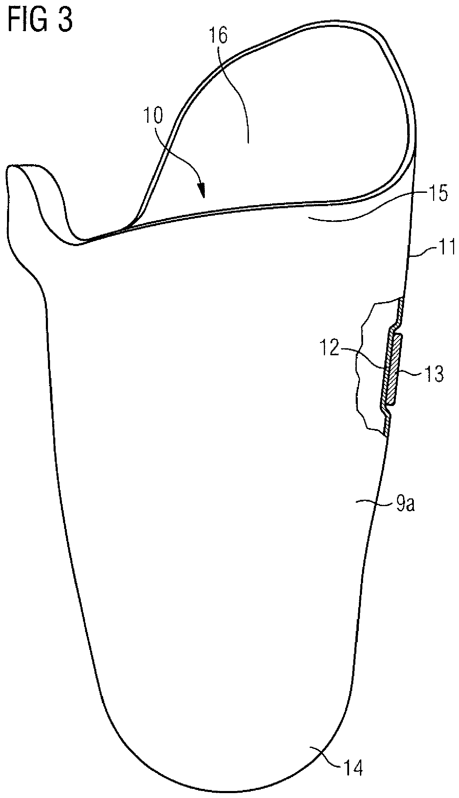

Figur 3 zeigt einen einschaligen Prothesenschaft 9a mit einer Auskragung 12 in einer Perspektivansicht. Der Prothesenschaft 9a weist eine hohle, konische Form auf und ist zur Aufnahme eines Oberschenkelstumpfes geeignet. Der Prothesenschaft 9a weist weiter eine Prothesenschaftwand 11 auf. Auf einer einer sich verjüngend zulaufenden Seite 14 abgewandten Seite 15 des Prothesenschafts 9a weist der Prothesenschaft 9a eine Öffnung 10 auf, von der an sich der Stumpfaufnahmebereich 16 bis zur sich verjüngend zulaufenden Seite 14 des Prothesenschafts 9a und innerhalb der Prothesenschaftwand 11 erstreckt. Somit weist der Prothesenschaft 9a eine ebenfalls konisch geformte Ausnehmung als Stumpfaufnahmebereich 16 auf. Im oberen Bereich des Prothesenschafts 9a, das heißt, in dem Bereich, der der Öffnung 10 zugewandt ist, weist die Prothesenschaftwand 11 eine zum Stumpfaufnahmebereich 16 hin gerichtete Auskragung 12 auf. Die Auskragung 12 weist eine Einrichtung 13 zur hydraulischen Regulierung der Tiefe der Auskragung 12 auf. Mit Tiefe ist hier das Maß gemeint, in welchem die Auskragung 12 in den Stumpfaufnahmebereich 16 hineinragt. Die Einrichtung 13 ist nicht von der Prothesenschaftwand 11 überdeckt sondern von außen sichtbar und zugänglich. Bei Betätigen der Einrichtung 13 wölbt sich die Einrichtung 13 mehr oder weniger in Richtung des Stumpfaufnahmebereichs 16 und vergrößert oder verringert dadurch die Tiefe der Auskragung 12.

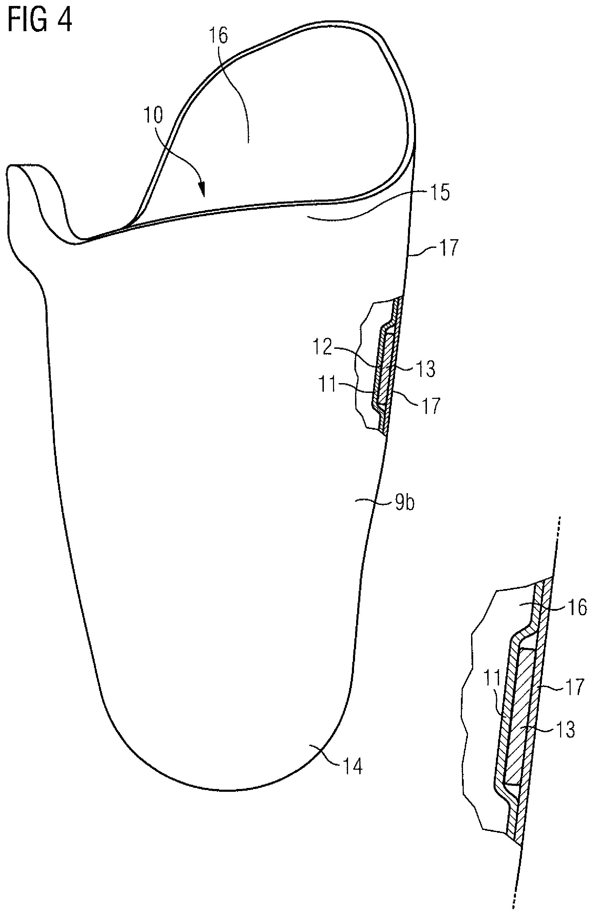

Figur 4 zeigt einen zweischaligen Prothesenschaft 9b, der ähnlich zu dem Prothesenschaft 9b aus Figur 3 ausgebildet ist und ebenfalls eine Auskragung 12 mit einer Einrichtung 13 zur hydraulischen Regulierung der Tiefe der Auskragung 12 aufweist. Im Unterschied zum Prothesenschaft 9a der Figur 3 weist der Prothesenschaft 9b eine zweischalige Prothesenschaftwand auf, wobei diese zum Stumpfaufnahmebereich 16 hin eine innere Wandung 11 und nach außen hin bzw. vom Stumpfaufnahmebereich weg gerichtet eine äußere Wand 17 aufweist. Dementsprechend ist die Einrichtung 13 zur hydraulischen Regulierung der Tiefe der Auskragung 12 zwischen der inneren Wandung 11 und der äußeren Wand 17 angeordnet, sodass die Einrichtung 13 von außen weder sichtbar noch zugänglich ist.

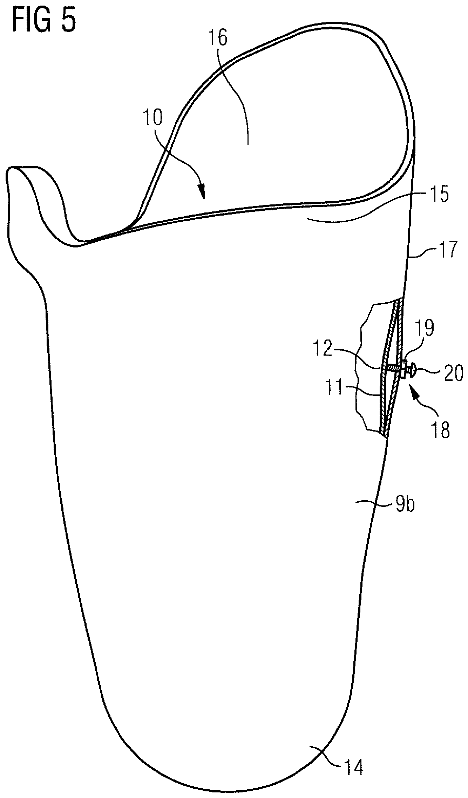

Figur 5 zeigt den Prothesenschaft 9b aus Figur 4, wobei dieser anstatt einer Einrichtung 13 zur hydraulischen Regulierung nun eine Einrichtung 18 zur mechanischen Regulierung der Tiefe der Auskragung 12 aufweist. Die Einrichtung 18 weist eine in etwa senkrecht zur inneren Wandung 11 und zur äußeren Wand 17 in ein Gewinde 19 eingeschraubte Schraube 20 auf. Das Gewinde 19 ist in der äußeren Wand 17 enthalten, sodass der Kopf der Schraube 20 nach außen gerichtet ist und von außen zur Betätigung der 20 zugänglich ist. Das dem Kopf der Schraube 20 abgewandte Ende der Schraube 20 berührt die innere Wandung 11 auf der dem Stumpfaufnahmebereich 16 abgewandten Seiten der inneren Wandung 11. Durch Ein- und Ausdrehen der Schraube 20 wird die innere Wandung 11 tiefer oder weniger tief zum Stumpfaufnahmebereich 16 hin gedrückt und somit die Tiefe der Auskragung vergrößert bzw. verkleinert.

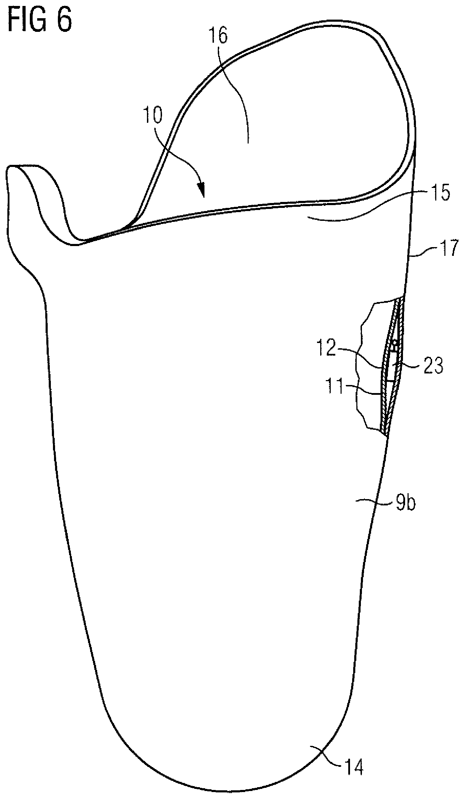

Figur 6 zeigt den Prothesenschaft 9b, wobei die Auskragung 12 ein TENS-Gerät 23 aufweist. Auch hier ist das TENS-Gerät 23 zwischen der inneren Wandung 11 und der äußeren Wand 17 angeordnet.

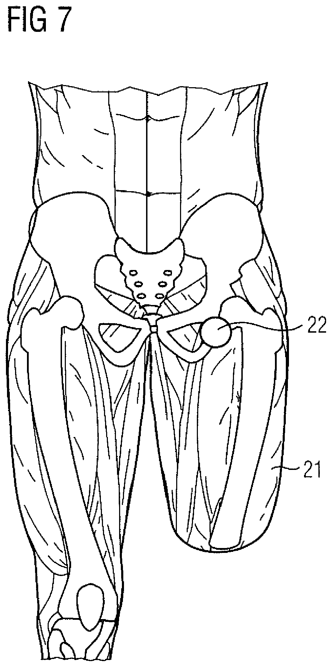

Figur 7 zeigt eine transparente schematische Darstellung eines menschlichen Oberschenkelstumpfes 21. Der Kreis 22 im oberen Bereich des dargestellten Oberschenkelstumpfes 21 kennzeichnet einen Ansatzpunkt für eine Wärme- und/oder Kühlkammer, die in einer Ausstülpung enthalten ist. Bei dem dargestellten Ansatzpunkt handelt es sich um eine zentrale Einströmstelle des arteriellen Blutes. Wird das Blut an dieser Stelle erwärmt bzw. abgekühlt, kann sich die Wärme bzw. Kühle aufgrund des Blutflusses schnell im gesamten Oberschenkelstumpf verteilen.

In einem Aspekt der vorliegenden Erfindung kann mindestens eine der Ausstülpungen der Prothese oder Orthese mindestens eine Kammer aufweisen.

In einem weiteren Aspekt der vorliegenden Erfindung kann mindestens eine der Ausstülpungen einen Permanentmagneten oder einen Elektromagneten zur Erzeugung eines Magnetfeldes beinhalten.

In einem weiteren Aspekt der vorliegenden Erfindung kann mindestens eine Kammer der mindestens einen Ausstülpung ein Piezoelement zur elektrischen und/oder mechanischen Stimulation beinhalten.

In einem weiteren Aspekt der vorliegenden Erfindung können in mindestens einer Kammer mindestens einer der Ausstülpungen Medikamente deponierbar oder deponiert sein, wobei die Medikamente über eine oder mehrere Öffnungen und/oder Poren in der mindestens einen Kammer an eine Oberfläche des Körperteils abgebbar sind.

In einem weiteren Aspekt der vorliegenden Erfindung kann mindestens eine der Ausstülpungen zur Beheizung und/oder Kühlung des Schaftes eingerichtet sein.

In einem weiteren Aspekt der vorliegenden Erfindung kann der Schaft von einem Schlauchsystem zur Heizung und/oder Kühlung durchzogen sein, das mit der mindestens einen Ausstülpung oder den Ausstülpungen wärmeleitend verbunden ist.

In einem weiteren Aspekt der vorliegenden Erfindung kann mindestens eine Kammer mindestens einer der Ausstülpungen als Wärmekammer zur Erwärmung von arteriellem Blut und /oder einer Kühlkammer zur Abkühlung arteriellen Blutes an einer zentralen Einströmstelle des arteriellen Blutes eingerichtet sein.

In einem weiteren Aspekt der vorliegenden Erfindung kann mindestens eine Kammer mindestens einer der Ausstülpungen ein Vibrationselement beinhalten.

In einem weiteren Aspekt der vorliegenden Erfindung kann mindestens eine Kammer mindestens einer der Ausstülpungen eine Füllung mit Feuchtigkeit aufsaugendem Material aufweisen.

In einem weiteren Aspekt der vorliegenden Erfindung kann in mindestens einer Kammer mindestens einer der Ausstülpungen ein elektronisch und/oder mechanisch wirkendes Entfeuchtungselement angeordnet sein.

In einem weiteren Aspekt der vorliegenden Erfindung kann in mindestens einer Kammer mindestens einer der Ausstülpungen mindestens ein Messgerät zur Messung von Körperzuständen, eines Blutdrucks, einer Strömungsgeschwindigkeit von Blut und/oder einer Körpertemperatur angeordnet sein.

In einem weiteren Aspekt der vorliegenden Erfindung kann in mindestens einer Kammer mindestens einer der Ausstülpungen mindestens ein Messgerät zur Erfassung von bionischen Daten, eines Oberflächendruckes an definierten Messpunkten, einer Schaftinnentemperatur und/oder eines Feuchtigkeitsgrades angeordnet sein.

In einem weiteren Aspekt der vorliegenden Erfindung kann mindestens eine der Ausstülpungen mindestens eine Elektrode zur Abnahme von myoelektrischen Impulsen aufweisen.

In einem weiteren Aspekt der vorliegenden Erfindung kann die innere Wandung vollständig mit Ausstülpungen überdeckt sein.

In einem weiteren Aspekt der vorliegenden Erfindung kann die innere Wandung und mindestens eine der Ausstülpungen das gleiche Material aufweisen und/oder mindestens eine der Ausstülpungen in die innere Wandung integriert sein.

In einem weiteren Aspekt der vorliegenden Erfindung kann die innere Wandung ein elastisches Material, ein Material mit einem weichen Shore-Grad oder ein rigides Material aufweisen.

In einem weiteren Aspekt der vorliegenden Erfindung kann eine Oberfläche mindestens einer der Ausstülpungen glatt, rau, adhäsiv, mit einem Relief und/oder mit Noppen versehen sein.

In einem weiteren Aspekt der vorliegenden Erfindung können die Noppen vollständig mit Material gefüllt oder hohl gestaltet sein.

In einem weiteren Aspekt der vorliegenden Erfindung kann die innere Wandung und die mindestens eine Ausstülpung unterschiedliche Materialien aufweisen.

In einem weiteren Aspekt der vorliegenden Erfindung kann mindestens eine der Ausstülpungen auf eine Oberfläche der inneren Wandung aufgeklebt sein.

In einem weiteren Aspekt der vorliegenden Erfindung kann mindestens eine der Ausstülpungen mit einer Pelotte gefüllt sein.

In einem weiteren Aspekt der vorliegenden Erfindung kann mindestens eine der Ausstülpungen temporär adaptierbar an der inneren Wandung angebracht sein.

In einem weiteren Aspekt der vorliegenden Erfindung kann mindestens eine Kammer mindestens einer der Ausstülpungen mindestens eine TENS-Elektrode aufweisen.

In einem weiteren Aspekt der vorliegenden Erfindung kann mindestens eine Kammer mindestens einer der Ausstülpungen einen Sensor für eine funktionelle Elektrostimulation aufweisen.

## Patentansprüche

1. Prothese oder Orthese mit einem mehrschaligen oder einschaligen Schaft (9a, 9b) zur Aufnahme eines Körperteils,
wobei der Schaft (9a, 9b) eine einen Körperteilaufnahmebereich (16) begrenzende innere Wandung (11) aufweist,
wobei die innere Wandung (11) den Körperteilaufnahmebereich (16) in einer Ebene vollständig umschließt und
wobei die innere Wandung (11) mindestens eine Ausstülpung (12) aufweist, **dadurch gekennzeichnet, dass** die mindestens eine Ausstülpung (12) temporär adaptierbar an der inneren Wandung (11) angebracht ist, sodass die mindestens eine Ausstülpung (12) umpositionierbar ist, und entlang eines Akupunktur-Meridians und/oder entlang einer Nervenbahn anordenbar ist.

2. Prothese oder Orthese nach dem vorhergehenden Anspruch, **dadurch gekennzeichnet, dass** die innere Wandung (11) eine Vielzahl von Ausstülpungen (12) aufweist, die entlang eines Akupunktur-Meridians und/oder entlang einer Nervenbahn anordenbar sind.

3. Prothese oder Orthese nach einem der vorhergehenden Ansprüche, **dadurch gekennzeichnet, dass** mindestens eine der Ausstülpungen (12) als Auskragung (12) oder als Vertiefung ausgebildet ist und geeignet ist eine Druck- und/oder Zugkraft auf einen Akupunkturpunkt auszuüben.

4. Prothese oder Orthese nach einem der vorhergehenden Ansprüche, **dadurch gekennzeichnet, dass** mindestens eine der Ausstülpungen (12) kuppelförmig, kantig, konvex, konkav, sternförmig, oval oder eckig ausgebildet ist.

5. Prothese oder Orthese nach einem der vorhergehenden Ansprüche, **dadurch gekennzeichnet, dass** der Schaft (9a, 9b) zylinderförmige oder konische Gestalt aufweist.

6. Prothese oder Orthese nach einem der vorhergehenden Ansprüche, **dadurch gekennzeichnet, dass** die Tiefe und/oder die Dicke mindestens einer der Ausstülpungen (12) pneumatisch oder hydraulisch oder mechanisch veränderbar ist.

7. Prothese oder Orthese nach dem vorhergehenden Anspruch, **dadurch gekennzeichnet, dass** im Schaft (9a, 9b) ein System miteinander kommunizierender Röhren zur hydraulischen oder pneumatischen oder mechanischen Regulierung der Tiefe und/oder Dicke der Ausstülpungen (12) angeordnet ist.

8. Prothese oder Orthese nach einem der beiden vorhergehenden Ansprüche, **dadurch gekennzeichnet, dass** zur Regulierung der Tiefe und/oder der Dicke der Ausstülpungen (12) ein Wechseldrucksystem vorgesehen ist, womit die Tiefe und/oder Dicke von einzelnen oder mehreren Ausstülpungen (12) abwechselnd erhöht oder verringert werden können.

9. Prothese oder Orthese nach einem der vorhergehenden Ansprüche, **dadurch gekennzeichnet, dass** mindestens eine der Ausstülpungen (12) mindestens eine Kammer aufweist.

10. Prothese oder Orthese nach dem vorhergehenden Anspruch, **dadurch gekennzeichnet, dass** mindestens eine Kammer mindestens einer der Ausstülpungen (12) einen Permanentmagneten oder einen Elektromagneten zur Erzeugung eines Magnetfeldes beinhaltet.

11. Prothese oder Orthese nach einem der beiden vorhergehenden Ansprüche, **dadurch gekennzeichnet, dass** die mindestens eine Kammer der mindestens einen Ausstülpung (12) ein Piezoelement zur elektrischen und/oder mechanischen Stimulation beinhaltet.

12. Prothese oder Orthese nach einem der drei vorhergehenden Ansprüche, **dadurch gekennzeichnet, dass** in mindestens einer Kammer mindestens einer der Ausstülpungen (12) Medikamente deponierbar oder deponiert sind, wobei die Medikamente über eine oder mehrere Öffnungen und/oder Poren in der mindestens einen Kammer an eine Oberfläche des Körperteils abgebbar sind.

## Claims

1. A prosthesis or orthosis with a multi-shell or single-shell shaft (9a, 9b) for receiving a body part,
wherein the shaft (9a, 9b) has an inner wall (11) delimiting a body-part receiving region (16),
wherein the inner wall (11) completely encloses the body-part receiving region (16) in one plane, and
wherein the inner wall (11) has at least one protuberance (12), **characterised in that**
the at least one protuberance (12) is attached temporarily in adaptable manner to the inner wall (11), so that the at least one protuberance (12) is repositionable, and can be arranged along an acupuncture meridian and/or along a neural pathway.

2. A prosthesis or orthosis according to the preceding claim, **characterised in that** the inner wall (11) has a multiplicity of protuberances (12) which can be arranged along an acupuncture meridian and/or along a neural pathway.

3. A prosthesis or orthosis according to one of the preceding claims, **characterised in that** at least one of the protuberances (12) is formed as a projection (12) or as an indentation, and is suitable for exerting a pressure force and/or tractive force on an acupuncture point.

4. A prosthesis or orthosis according to one of the preceding claims, **characterised in that** at least one of the protuberances (12) is designed in a dome-shape, in edged, convex, concave, star-shaped or oval manner, or with corners.

5. A prosthesis or orthosis according to one of the preceding claims, **characterised in that** the shaft (9a, 9b) is of cylindrical or conical form.

6. A prosthesis or orthosis according to one of the preceding claims, **characterised in that** the depth and/or the thickness of at least one of the protuberances (12) is changeable pneumatically or hydraulically or mechanically.

7. A prosthesis or orthosis according to the preceding claim, **characterised in that** a system of communicating tubes for hydraulic or pneumatic or mechanical regulation of the depth and/or thickness of the protuberances (12) is arranged in the shaft (9a, 9b).

8. A prosthesis or orthosis according to one of the preceding two claims, **characterised in that** an alternating-pressure system is provided for regulating the depth and/or the thickness of the protuberances (12), with which system the depth and/or thickness of individual or a plurality of protuberances (12) can be increased or reduced in alternation.

9. A prosthesis or orthosis according to one of the preceding claims, **characterised in that** at least one of the protuberances (12) has at least one chamber.

10. A prosthesis or orthosis according to the preceding claim, **characterised in that** at least one chamber of at least one of the protuberances (12) contains a permanent magnet or an electromagnet for generating a magnetic field.

11. A prosthesis or orthosis according to one of the preceding two claims, **characterised in that** the at least one chamber of the at least one protuberance (12) contains a piezo element for electrical and/or mechanical stimulation.

12. A prosthesis or orthosis according to one of the preceding three claims, **characterised in that** medicaments are or can be deposited in at least one chamber of at least one of the protuberances (12), the medicaments being able to be dispensed onto a surface of the body part via one or more openings and/or pores in the at least one chamber.

## Revendications

1. Prothèse ou orthèse avec une tige multicoque ou monocoque (9a, 9b) pour le logement d'un membre,
la tige (9a, 9b) comprenant une paroi interne (11) délimitant une partie de logement de membre (16),
la paroi interne (11) entourant complètement la partie de logement de membre (16) dans un plan et
la paroi interne (11) comprenant au moins une protubérance (12), **caractérisée en ce que** l'au moins une protubérance (12) peut être montée de manière temporairement adaptable sur la paroi interne (11), de façon à ce que l'au moins une protubérance (12) puisse être déplacée et être disposée le long d'un méridien d'acupuncture et/ou le long d'une voie nerveuse.

2. Prothèse ou orthèse selon la revendication précédente, **caractérisée en ce que** la paroi interne (11) comprend une pluralité de protubérances (12) qui peuvent être disposées le long d'un méridien d'acupuncture et/ou le long d'une voie nerveuse.

3. Prothèse ou orthèse selon l'une des revendications précédentes, **caractérisée en ce que** l'au moins une des protubérances (12) est conçue comme une saillie (12) ou comme un creux, et est conçue pour exercer une force de pression et/ou de traction sur un point d'acupuncture.

4. Prothèse ou orthèse selon l'une des revendications précédentes, **caractérisée en ce qu'**au moins une des protubérances (12) présente une forme de coupole, une forme anguleuse, convexe, concave, une forme d'étoile, ovale ou angulaire.

5. Prothèse ou orthèse selon l'une des revendications précédentes, **caractérisée en ce que** la tige (9a, 9b) présente une forme cylindrique ou conique.

6. Prothèse ou orthèse selon l'une des revendications précédentes, **caractérisée en ce que** la profondeur et/ou l'épaisseur d'au moins une des protubérances (12) peut être modifiée de manière pneumatique, hydraulique ou mécanique.

7. Prothèse ou orthèse selon la revendication précédente, **caractérisée en ce que**, dans la tige (9a, 9b), est disposé un système de tubes communiquant entre eux pour la régulation hydraulique, pneumatique ou mécanique de la profondeur et/ou de l'épaisseur des protubérances (12).

8. Prothèse ou orthèse selon l'une des deux revendications précédentes, **caractérisée en ce que**, pour la régulation de la profondeur et/ou de l'épaisseur des protubérances (12), un système de pression variable est prévu, qui permet tour à tour d'augmenter ou de réduire la profondeur et/ou l'épaisseur de certaines ou de plusieurs protubérances (12).

9. Prothèse ou orthèse selon l'une des revendications précédentes, **caractérisée en ce qu'**au moins une des protubérances (12) comprend au moins une chambre.

10. Prothèse ou orthèse selon la revendication précédente, **caractérisée en ce qu'**au moins une chambre d'au moins une des protubérances (12) contient un aimant permanent ou un électro-aimant pour la production d'un champ magnétique.

11. Prothèse ou orthèse selon l'une des deux revendications précédentes, **caractérisée en ce que** l'au moins une chambre de l'au moins une protubérance (12) contient un élément piézo-électrique pour la stimulation électrique et/ou mécanique.

12. Prothèse ou orthèse selon l'une des trois revendications précédentes, **caractérisée en ce que**, dans au moins une chambre d'au moins une des protubérances (12), des médicaments peuvent être déposés ou sont déposés, les médicaments pouvant être administrés par l'intermédiaire d'une ou de plusieurs ouvertures et/ou pores dans l'au moins une chambre sur une surface du membre.
